(19) Europäisches Patentamt

European Patent Office

Office européen des brevets

(11) EP 0 477 434 B2

(12) NEW EUROPEAN PATENT SPECIFICATION

(45) Date of publication and mention
of the opposition decision:
**14.11.2001 Bulletin 2001/46**

(45) Mention of the grant of the patent:
**28.02.1996 Bulletin 1996/09**

(51) Int Cl.[7]: **A61B 5/04**, G01R 33/035,
H01Q 3/26

(21) Application number: 90124050.7

(22) Date of filing: **13.12.1990**

(54) **Analysis of biological signals using data from arrays of sensors**

Analyse von biologischen Signalen unter Verwendung von Daten von Sensorarrays

Analyse de signaux biologiques utilisant des données des réseaux de détecteurs

(84) Designated Contracting States:
**AT BE CH DE DK ES FR GB GR IT LI LU NL SE**

(30) Priority: **24.09.1990 US 585867**

(43) Date of publication of application:
**01.04.1992 Bulletin 1992/14**

(73) Proprietor: **BIOMAGNETIC TECHNOLOGIES, INC.**
**San Diego, CA 92121-3719 (US)**

(72) Inventors:
• **Robinson, Stephen E.**
**San Diego, CA 92127 (US)**
• **Black, William C., Jr.**
**Del Mar, CA 92014 (US)**

(74) Representative:
**Dreiss, Fuhlendorf, Steimle & Becker**
**Patentanwälte,**
**Postfach 10 37 62**
**70032 Stuttgart (DE)**

(56) References cited:
**US-A- 4 417 591**

• **PROCEEDINGS OF THE ANNUAL INTERNATIONAL CONFERENCE OF THE IEEE ENGINEERING IN MEDICINE AND BIOLOGY SOCIETY vol. 11, no. 2, 12 November 1989, SEATTLE(US) pages 702 - 704; J.J.WESTERKAMP ET AL.: 'ADAPTIVE ESTIMATION AND DETECTION OF EVOKED POTENTIALS'**
• **PROCEEDINGS OF THE ANNUAL INTERNATIONAL CONFERENCE OF THE IEEE ENGINEERING IN MEDICINE AND BIOLOGY SOCIETY vol. 11, no. 4, 12 November 1989, SEATTLE(US) pages 1256 - 1258; C.W. CROWLEY ET AL.: 'NEW APPROACHES TO SOURCE LOCALISATION IN MEG'**
• **ELECTRO MEDICA (SIEMENS), Vol. 57, no. 1, 1989, Erlangen DE, pages 2-7; F.GUDDEN ET AL. "Ein Vielkanalsystem zur Biomagnetischen Diagnostik in Neurologie und Kardiologie: Prinzip, Methode und erste Ergebnisse"**
• **GEC JOURNAL OF RESEARCH (Incorporating Marconi Review) vol.3, no.1, 1985, great Baddow Chelmsford GB, pages 34-45; B.WARDROP: "THE ROLE OF DIGITAL PROCESSING IN RADAR BEAMFORMING", page 35, Chapter 2 "Algorithms and Architecture"**
• **IEEE Engineering in Medicine & Biology Society 11th Annual International Conference (1989), pages 1256-1258; C.W. Crowley et al., "New Approaches to Source Localization in MEG"**
• **Advances in Biomagnetism, Plenum Press New York and London (1989), pages 599-602; S.E. Robinson, "Theory and Properies of Lead Field Synthesis Analysis"**

**Description**

BACKGROUND OF THE INVENTION

This invention relates to the measurement of electromagnetic signals originating in the human body, and, more particularly, to the measurement of magnetic and/or electrical fields originating from brain activity.

The present invention was made in part under US government contract F33615-89-C-0577. The government may have rights in this invention.

The human body produces magnetic fields that can be measured externally by a biomagnetometer. The research on correlating biomagnetic fields with various states of health, disease and abnormality is underway, but sufficient information is available to demonstrate that certain emitted biomagnetic fields are associated with conditions such as epilepsy. Present medical studies are investigating the nature of the normal and abnormal magnetic fields of the brain, and seeking to correlate those fields with brain functions and patient health.

It is well established that certain physically identifiable locations in the brain are responsible for specific types of activities and functions. It is therefore important to correlate the measured biomagnetic field with the particular location in the brain which produces the field. Such a correlation is important to understanding the mechanism by which disease and disorder arise, and also to the treatment of the problem.

Correlating the spontaneous measurement taken by an external array of magnetic or electrical sensors with brain activity at a specific location within the brain is difficult, primarily because other areas of the brain continue to function and produce their own magnetic and electrical fields, even as a measurement is being taken with the intent of measuring activity at a specified location, and because the measurement sensors and instrumentation produce noise that may be of the same magnitude as the signals to be measured. It is not easily determined whether a particular signal measured externally originates at the selected location, other locations, or jointly at the selected location and other locations, or in fact is a manifestation of instrument noise. At the present time, there is a good deal of reliance on averaging multiple occurrences of magnetic and/or electric signals synchronously with external events such as a stimulus, to isolate the origin of particular magnetic signals.

Moreover, it is difficult to develop data from spontaneous brain activity, having a high signal to noise ratio, that can reliably be said to originate at a selected location in the brain. Having such a capability would be extremely useful, because it would permit studies of neurological disorders associated with epilepsy, stroke, and head injury, for example, and even direct physiological studies of some of the most basic phenomena of life, such as attention and boredom, mental disorders, language comprehension and expression, and response to external stimuli.

An important step in correlating external measurements with the specific locations of internal events is disclosed in US patent 4,793,355, which provides a methodology for automatically tracking the position of the sensors in respect to the position of the head of the patient. When used in conjunction with the known spatial sensitivity profile of the detector and either an external stimulus or synchronization with voluntary activity, this approach gives important information about the internal origin of the externally measured signal. This technology, by itself, is limited in its resolution of location and nature of the source, because of various types of noise and the continued operation of other brain functions as measurements are taken. It is also limited in its ability to investigate spontaneous, non-evoked brain activity.

One approach to analysis of biological signals recorded with a multi-channel sensor system is reported by J. J. Westerkamp et al in Proceedings of the Annual International Conference of the IEEE Engineering and Medical Society, Volume II, part 2/6 (1989), pages 702 - 703. In that work, the output of each of the channels in the multi-channel sensor system is multiplied by a weighting factor using an adaptive algorithm for determining the weighting factors. The algorithm and method was designed in order to minimize distortion in the desired signal and to reduce the noise from interfering EEG signals. While this approach can improve the quality of the resultant signal in general, it does not address the goal of determining the locations of the sources of the biological signals recorded. In Proceedings of the Seventh International Conference on Biomagnetism, Plenum Press, New York, Advances in Biomagnetism, Theory and Properties of Lead Field Synthesis Analysis, pages 599 to 602, it is outlined that a signal analysis process was developed for combining simultaneous measurements made from multiple physical sensors into a synthetic virtual sensor having spatially selective and directional properties.

SUMMARY OF THE INVENTION

The present invention provides an approach for analyzing electromagnetic signals, such as magnetic and electrical fields, produced by the body. It permits selection of the location within the body from which the signals are to be examined, and an excellent characterization of the signals themselves with an optimized signal to noise ratio. The approach can be practiced with existing hardware, or can utilize apparatus that is specially designed for the purpose.

The present invention further provides an approach for utilizing multiple electromagnetic sensors of an array in combination to obtain better information about the functioning of an electromagnetic source within the body, than could be obtained from the uncombined data of the individual sensors of the array. It provides an approach for mathematically combining the outputs of the sensors of the array in a manner that effectively defines a virtual sensor which is optimized for measuring a particular location within the body, based upon the characteristics of the sensors, the sources, and the body, and their relative locations. The outputs of the individual sensors are weighted and added together to define the virtual sensor output, and different virtual sensors can be defined from a fixed sensor array.

In accordance with the invention, a process for sensing and evaluating signals emanating from the brain of a subject comprises the steps of providing an array of field sensors disposed external to a body of a subject at known locations; measuring a signal strength detected by each of said sensors; multiplying the signal strength measured by each of said sensors times a weighting coefficient for that sensor, selecting said weighting coefficient to focus the sensitivity of the array of field sensors on a selected location within the body of the subject, to determine a virtual sensor contribution for each of said sensors; and adding together the virtual sensor contribution of each of said sensors, to define a virtual sensor signal.

The present invention utilizes the combined response data gathered by each one of an array of sensors external to the body. The sensors can be sensitive to magnetic fields or electrical fields, and the term "electromagnetic" is used herein as a generic descriptor for signals, detectors, or the like to encompass both types of phenomena. The combined data can be treated to attain an optimized characterization of the signal produced from a selected location in the brain or other source. By combining the same response data of the sensors in different ways, simultaneous occurrences in different parts of the body can be understood by themselves and in relation to each other. The present approach can be used to measure any portion of the body from which electromagnetic signals originate, including but not limited to the brain, the heart, muscle tissue, nerves, etc.

To calculate the virtual sensor signal corresponding to an electromagnetic field generated at a selected location in the brain, the signal strength measured by each sensor at a point in time is multiplied by a weighting coefficient determined for that sensor, and then all the products of signal strength and weighting coefficient are added together. The weighting coefficient is calculated either from a formulated mathematical model, or, preferably, from the actual measurements of the sensors, together with the relative positions and orientation of the sensors and the selected location in the body, and the spatial sensitivity of the sensor, known as its lead field. From this last component comes the term used to describe this type of analysis, "lead field synthesis" ("LFS").

One desirable feature of this approach is that the nature of the virtual sensor, attained from the weighted combination of the signals of the individual physical sensors, can be changed to tune the response of the virtual sensor to different locations, using the same measured data. Thus, with a first set of weighting coefficients, the virtual sensor is directed to sample a first location in the body, and with a second set of weighting coefficients, the virtual sensor is directed to sample a second location in the body, in both cases using one set of actual measurement data from the physical array of sensors.

Another desirable feature is that the virtual sensor may be tuned for an optimal signal to noise ratio for a particular location, again through the mathematical model that provides the weighting coefficients. The signal to noise ratio is improved by making the virtual sensor less sensitive to brain activity in regions that are not of interest, or by reducing the effective instrument noise, including unwanted environmental signals. This capability permits the virtual sensor to utilize sophisticated signal acquisition and processing theories already known but never applied to the understanding of the body. The virtual sensor provides a means of combining data from both magnetic and electrical field sensors into a virtual sensor whose measurement units are source strength. This combination renders the measurement of activity independent of the type of sensor or its location.

The virtual sensor signal can be interpreted as the amount of the signal strength measured by all sensors simultaneously which could have originated at a specified source within the brain, to which the virtual sensor is directed.

The approach of the invention may be used with existing biomagnetometers such as that disclosed in US patent 4,793,355, and available commercially from Biomagnetic Technologies, Inc., San Diego, California. These biomagnetometers provide a large number of SQUID-type sensors in an array placed at various positions around the head of the subject under study. The response signal of each sensor is recorded separately, and combined using the approach just described to calculate a virtual sensor response. However, it is possible that other arrays of sensors may be devised that are optimally placed for utilization of the lead field synthesis methodology. In particular, it is expected that larger arrays of electromagnetic sensors, measuring magnetic and electrical signals, will provide even more precise information about the magnitude of signals originating at a particular location within the body.

Other features and advantages of the present invention will be apparent from the following more detailed description of the preferred embodiment, taken in conjunction with the accompanying drawings, which illustrate, by way of example, the principles of the invention.

BRIEF DESCRIPTION OF THE DRAWINGS

Figure 1 is a schematic representation of the sensitivity of a single-loop magnetic sensor;

Figure 2 is a schematic diagram of the magnetic field of a source in the brain;

Figure 3 is a schematic diagram of the placement of sensors to measure the magnetic field of a source in the brain;

Figure 4 is a schematic diagram of the placement of sensors to measure the magnetic field of different sources in the brain;

Figure 5 is a schematic diagram of the manner of combining the measurements of an array of sensors;

Figure 6 is a graph of signal intensity as a function of time for 37 channels of biomagnetic data, 2 channels of EEG data, and the result of a spatial filtering calculation;

Figure 7 is a graph of signal intensity as a function of time for 1 channel of biomagnetic data, 2 channels of EEG data, and the result of a spatial filtering calculation;

Figure 8 is a plot of image intensity as a function of position within the brain at a moment in time during an epileptic episode.

Figure 9 is a block diagram for a model-based process for performing lead field synthesis for brain signals;

Figure 10 is a block diagram for an augmented process for performing lead field synthesis for brain signals;

Figure 11 is a graph of the measured signal as a function of time for a sensor positioned proximate to the head of a person; and

Figure 12 is a graph of a synthesized virtual sensor signal as a function of time.

DETAILED DESCRIPTION OF THE PREFERRED EMBODIMENT

The approach of the present invention can be used in conjunction with measurements of magnetic and electrical signals from any and all parts of the body. Signals produced from the brain are of great interest, and the preferred embodiment is directed toward this application. The methodology, however, is more generally applicable.

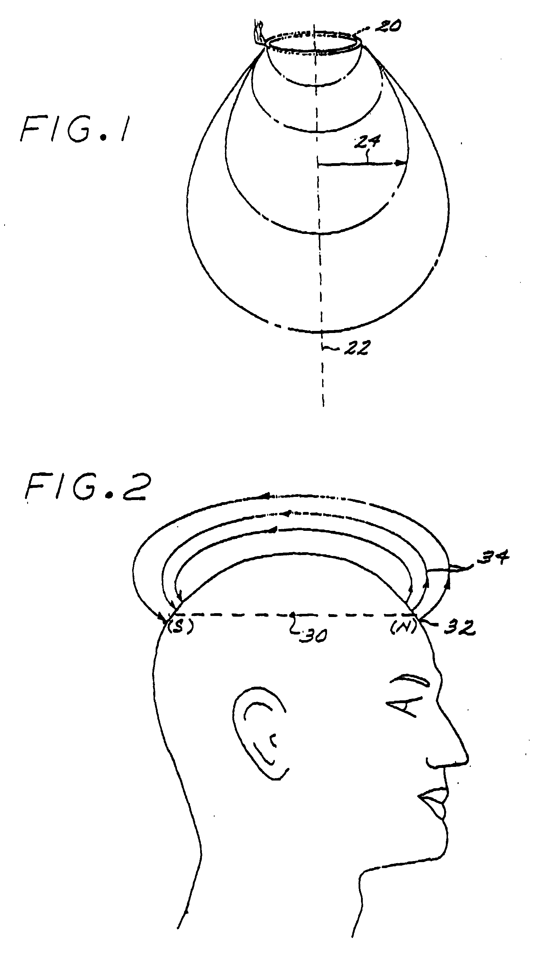

Sensors used to detect magnetic or electrical signals generated in the brain, like all such sensors, are directional in nature and have a sensitivity to signals that depends on the physical position of the sensor relative to the location whereat the signal originates, the orientation of the sensor, and the nature of the source of the signal. Generally, as illustrated in Figure 1 for a single loop magnetic field sensor 20, the further the origin of the signal from the sensor, the less sensitive the sensor is to the signal. Sensitivity, as represented by the curves of the figure extending below the sensor 20, decreases with increasing axial distance along the axis 22 of the loop, and also with radial distance 24 from the axis 22. The pattern of Figure 1 is illustrative of such sensitivity variations.

Another aspect of the design of sensors is that the larger the loop of the sensor, the more sensitive it should be to measuring small uniform magnetic fields, because more magnetic flux lines can pass through the loop. However, the placement of the sensor also influences its sensitivity to particular signals.

If one knows beforehand the location of the origin and the orientation of a specific signal to be measured, an optimal design and placement for the sensor used to measure the signal can be selected. However, such is not possible for a general-purpose approach, nor is it possible where signals from many locations are to be measured simultaneously or nearly simultaneously.

The following example illustrates the principles of sensor selection for a known source in the brain. The brain generates magnetic signals as a result of electrical currents that originate within the brain. Figure 2 illustrates the magnetic field produced by a source 30 within the head 32 of the person. The field originating at source 30 is produced by a neural current passing in the direction perpendicular to the page, along a group of neurons located at source 30. Magnetic field lines 34 emerge from the skull to the external environment, where a sensor may be located.

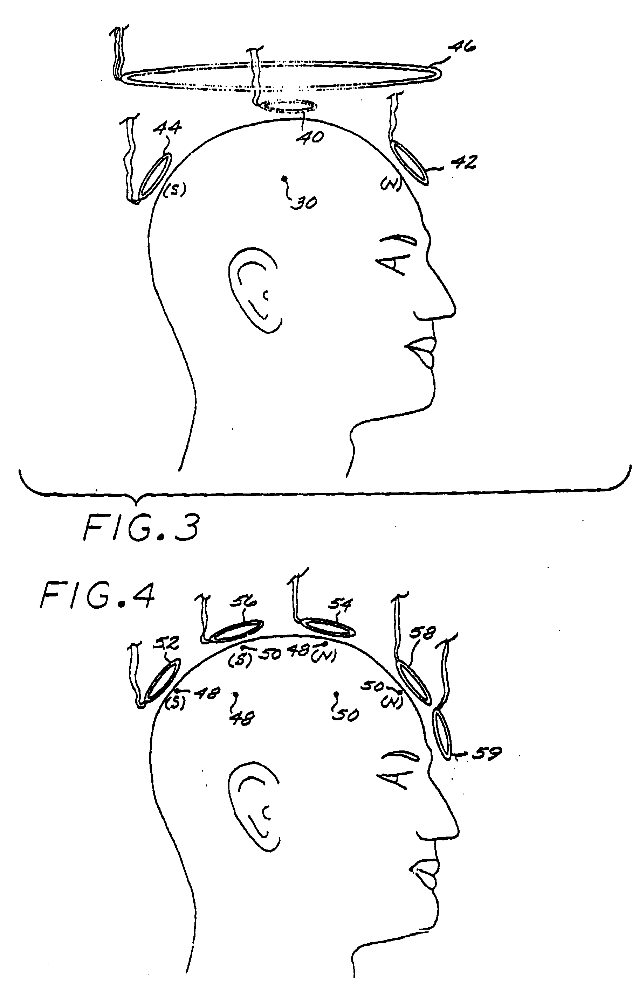

Figure 3 shows the same source 30, but with some external loop sensors positioned to measure the magnetic field. A sensor 40, positioned directly over the current source 30, registers no signal because no magnetic field lines 34 (omitted from Figure 3, but the same as in Figure 2) pass through the loop of the sensor 40. A sensor 42 is located above the N pole, and registers a maximum positive signal. A sensor 44 is located above the S pole, and registers a maximum negative signal. A much larger sensor 46, symmetrically located with respect to the N pole and the S pole, although more sensitive to uniform magnetic fields, registers no signal because the net flux through the loop of the sensor is zero.

The actual problem faced in making measurements of signals originating in the brain is that, unlike the example illustrated in Figures 2 and 3, the location and orientation of the signal source is not known. Figure 4 illustrates the nature of the sensing problem where there are two sources, and also the origin of the present analytical approach. Where there are two sources 48 and 50, sensors 52 and 54 placed over the 48N and 48S poles for source 48 will be most sensitive to that source, but will also detect some signal due to the other source 50. Likewise, sensors 56 and 58 placed over the 50N and 50S poles for source 50 will be most sensitive to the source 50, but will also detect some signal due to the other source 48. A sensor 59 may be placed so that it does not provide much information about either of the sources 48 and 50.

Thus, if there were a way to utilize the readings of sensors 56, 58, and 59 so as to render the measurements of sensors 52 and 54 insensitive to source 50, an optimal measurement of source 48 could be obtained. This result can be accomplished by subtracting the interfering signal arising from source 50, as best detected by sensors 56 and 58, from the signals detected by sensors 52 and 54, which respond maximally to the desired source 48. Thus, by knowing the proportions and sign with which to combine the signals from all sensors, an optimal measurement can be obtained for a specified source, and interference from unwanted sources can be suppressed. A similar principle holds for obtaining an optimal measurement from source 50 by utilizing the sensors 56 and 58, which convey information primarily about source 50, and sensors 52, 54, and 59, which convey information primarily from the source 48.

Even for the relatively simple example of Figure 4, the problem of sensing becomes more difficult if none of the sensors are located at the optimal positions for measuring the field of a selected source location and orientation. If none of the sensors of the array 52, 54, 56, 58, and 59 are optimally placed to measure a selected source, then some method must be chosen to utilize the data from some or all of the sensors in order to measure that source.

In principle, the sensitivity of the array of sensors 52, 54, 56, 58, and 59 can be adjusted or "tuned" to be optimally sensitive to signals originating from sources in the brain of particular locations and orientations. The approach for achieving such tuning is apparent from the very simple example of Figure 4, where the locations and orientations of the sources 48 and 50 were specified. The approach is not apparent for the more complex case of millions of sources of signals in the brain having locations and orientations that are not known beforehand, where a relatively small number of sensors are present in the array of sensors, and where there is no prior knowledge that the sensors are optimally placed for measuring a signal.

The present invention provides a technique for utilizing the data produced by an array of sensors to synthesize a "virtual" sensor that is optimally sensitive to a selected source within the body.

Considering both the spatial sensitivity of the sensor, as illustrated in Figure 1, and the geometric realities of sensor placement, as illustrated in Figures 2-4, it is apparent that the needs of a general measurement approach result in some apparently contradictory hardware requirements. Small diameter sensors are desirable to achieve good resolution of signals from shallow locations close to the skull, while large diameter sensors are desirable to achieve measurement of deep sources. Sensors should somehow be effectively capable of being repositioned to measure some sources, while remaining stationary to measure those sources for which they are optimally positioned.

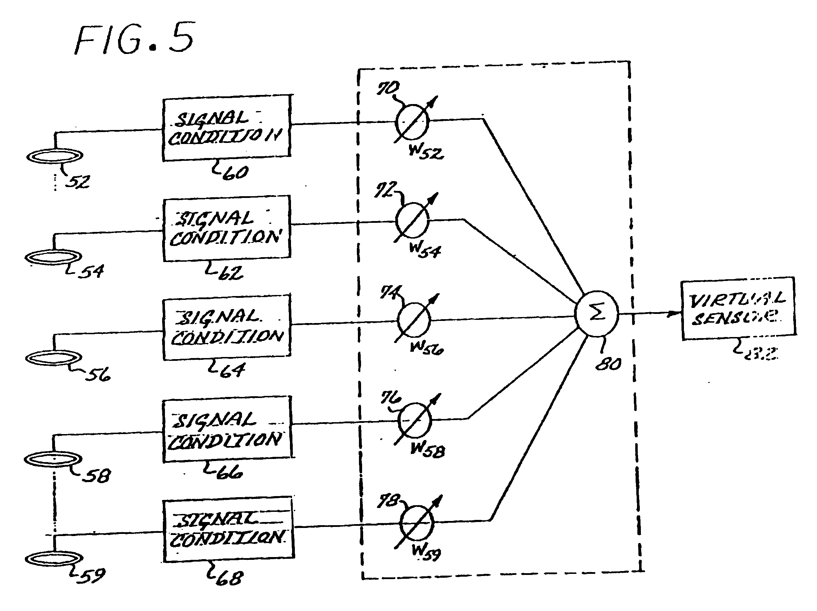

The present approach permits these apparently contradictory requirements to be met by utilizing all of the data from the multiple sensors to arrive at a single scalar number that is representative of the magnetic signal produced from a selected location, without physically moving or replacing any of the sensors. As illustrated in Figure 5, each response signal strength measured by a sensor such as sensors 52, 54, 56, 58, and 59 is first detected and conditioned by filtering and amplifying as necessary, in a signal conditioner depicted as element 60, 62, 64, 66, and 68, respectively. Such detection and signal conditioning apparatus is well known in the art, and is used for existing sensors.

The conditioned signal for each sensor is then multiplied by its own particular signed weighting coefficient, here illustrated as the weighting coefficients $w_{52}$, $w_{54}$, $w_{56}$, $w_{58}$, and $w_{59}$, respectively, in individual multiplying operations 70, 72, 74, 76, and 78. The sign (plus or minus) of the weighting coefficient is taken into account in performing the multiplying operation. A negative weighting coefficient results in a scaled product of polarity opposite to that of the original signal. Each of the resulting arithmetic products is supplied to an adder 80, which adds them together for the five sensors 52, 54, 56, 58, and 59. The resulting sum is supplied as a scalar signal termed a virtual sensor 82. In the preferred approach, the signals from the signal conditioners 60, 62, 64, 66, and 68 are digitized and supplied to a digital computer, which performs the multiplier functions 70, 72, 74, 76, and 78, and the adding function 80, in digital format. (The preceding example dealt with only five sensors, but the present invention is not so limited. Preferably, much larger arrays of sensors are used to improve resolution of sources, and the present approach has been tested with data taken from an array of 37 sensors.)

The effect of the summing of weighted signals approach may be understood with reference to Figure 4. With regard to the measurement of the magnetic field emanating from the source 48, the sensor 59 would gather almost no useful information, and in fact measure mostly brain noise relative to the source 48. The weighting coefficients of sensor 59 would be a small value, The sensors 52 and 54, on the other hand, gather a great deal of information about the source 48, and their weighting coefficients would be large and of opposite sign. The sensors 56 and 58 present the most difficult aspect of the analysis, since they provide some useful information about the source 48, but also a good deal of brain noise resulting from other sources, such as the source 50 (which must be considered noise when one attempts to study the signal produced by the source 48). The weighting coefficients of the sensors 56 and 58 would be of opposite sign to that of the sensors 52 and 54.

Using the same information gathered from sensors 52, 54, 56, 58, and 59, optimized information about the source 50 can be synthesized. Once again, the weighting coefficient for the sensor 59 would be small, because it contributes little useful information to analyzing the source 50. However, in this case the weighting coefficients for the sources 56 and 58 would be large, since they are optimally placed for measuring source 50. The weighting coefficients for the sources 52 and 54 would be of opposite sign, because they gather some useful information but also reflect brain noise from source 48 (which in this case is considered noise with respect to the source 50 of interest) and other sources.

The information gathered by the array of sensors 52, 54, 56, 58, and 59 may be combined with yet other weighting coefficients to measure other sources not illustrated in Figure 4. Although in the hypothesized examples of Figure 4, the sensor 59 contributed little of use in measuring either source 48 or 50, for other sources in different locations the sensor 59 may become the most highly weighted sensor and some or all of the sensors 52, 54, 56, and 58 may have lower weights and different signs.

Only one set of actual physical measurements using the sensors 52, 54, 56, 58, and 59 is made to yield information about the sources 48 and 50. The analytical approach permits those measurements to be combined in several ways, by using different weighting coefficients, to obtain the information about the individual sources. It is therefore possible, when the computer is used for the computations, to make one measurement using the sensors, store the measured values in the computer, and then perform a large number of computations using that measured and stored experimental data to deduce information about the magnetic signals produced by different sources at a single instant in time. Subsequent experimental results for the sensors are similarly stored and processed, permitting an understanding of the operation of each of the sources over time, both as to their individual operation and as to any interactions that may occur between sources.

The examples just presented illustrate the philosophical basis of lead field synthesis. However, they are purposely made to be not complex to illustrate the principles and possibilities of the approach. The vector nature of the sources 48 and 50, the type of source operating, and the magnetic permeability and electrical conductivity of the air, skull, and brain matter have not been discussed. Nor have the exact sensor locations and orientations, and the detailed geometry of the brain been incorporated into this qualitative description.

The more general implementation of lead field synthesis requires a mathematical study of the interaction of electromagnetic sources and sensors. The response of a magnetic sensor to a current source in the brain is directly proportional to the current I induced in the sensor coil,

$$I = K \int \vec{G}(\vec{r}) \cdot \vec{H}(\vec{r}) \, dV$$

where $\vec{G}(\vec{r})$ and $\vec{H}(\vec{r})$ are the sensor lead field and the magnetic field generated by the source current at position $\vec{r}$. $\vec{G}(\vec{r})$ is a characteristic of the sensor, and $\vec{H}(\vec{r})$ is calculated from the source and media properties, as will be described subsequently.

If the sensor output of an array of sensors is represented as a vector $\vec{s}$ and the weight to be accorded each sensor is represented by a vector $\vec{w}$ of equal dimension, the scalar output signal V is the dot product of the weight and sensor output vectors:

$$V = \vec{w} \cdot \vec{s}$$

Thus, the lead field of a virtual sensor defined by the summation of the weighted outputs of an array of sensors is

$$G_v = \Sigma_i w_i G_i$$

For an array of M sensors and a model, $\Omega$, of all potential sources of brain signal, including a target source, the Gram matrix is computed as

$$\Gamma_{ij} = \int_\Omega \vec{G}_i \vec{G}_j \, d\Omega$$

where (i,j, = 1, ..., M) and $G_i$ is the Green's function for the response of the ith detector at the integration point in source space. By specifying the Green's functions for the desired "target" source, $\vec{G}_T$ , a set of coefficients $\vec{w}$ can be calculated as

$$\vec{W} = \Gamma_{ij}^{-1}\vec{G}_T \Omega.$$

One approach to solving this problem is to formulate a mathematical model of the brain or other portion of the body being measured, and is discussed subsequently. A preferred approach is to use the measurements themselves to develop the weighting factors.

The more potential sources relative to the number of sensors, the more difficult it is to distinguish a single signal, leading to a "blurriness" of the solution. However, the scope of the problem may be reduced, leading to a more precise solution, by limiting the number of sources to those actually operating during any particular measurement period of time.

A covariance matrix $C_{ij}$ may be written as

$$C_{ij} = \frac{1}{T}\int_T (x_i(t) - \bar{x}_i)(x_j(t) - \bar{x}_j)dt$$

where $X_i$ is the signal received by a sensor during a period of time dt and $\bar{x}_i$ is the mean signal of that sensor during integration time T. Other than a scale factor, the covariance matrix is a "local" (in a temporal sense) estimator of the Gram matrix. The two matrices contain similar information, the Gram matrix from the source point of view and the covariance matrix from the sensor point of view. They each represent the interrelationship of the responses or lead fields of each of the sensors to each of the sources.

The scale factor relating the covariance matrix to the Gram matrix is calculated by requiring that the transfer function at the target location must be unity. That is,

$$\vec{W} \cdot \vec{G}_T = 1.$$

The unscaled lead field synthesis coefficients, alpha, are:

$$\overrightarrow{alpha} = [C_{ij}]^{-1}[\vec{G}_T].$$

Using the definition of transfer function, the scale factor relating the covariance matrix to the Gram matrix is

$$\vec{G}_T \cdot \overrightarrow{alpha}.$$

The scaled lead field synthesis coefficients are therefore

$$\vec{W} = \overrightarrow{alpha}/\vec{G}_T \cdot \overrightarrow{alpha}$$

or alternatively stated

$$\vec{W} = [C_{ij}]^{-1}[\vec{G_T}]/[\vec{G_T}] \cdot [C_{ij}]^{-1}[\vec{G_T}].$$

This result is important, because it means that a statistical measure of the body field signal, the covariance matrix $C_{ij}$, can be combined with generalized inverse theory to compute optimal brain noise reduction coefficients $\vec{W}$.

This approach has been assessed with actual test results for a 37-channel biomagnetometer, using an approach like that just discussed but with 37 sensors rather than five. The relative physical locations of the sensors with respect to sites on the surface of the head and inside the brain matter may be determined in real time, simultaneous with the field measurements, in the manner illustrated in US patent 4,793,355 or other acceptable manner. In general, a series of vectors defines points and locations with respect to each other, and the structure disclosed in the '355 patent permits such vector location information to be determined and stored by measurements in real time. The geometry of the outer surface of the head can also be measured and recorded in the manner disclosed in the '355 patent.

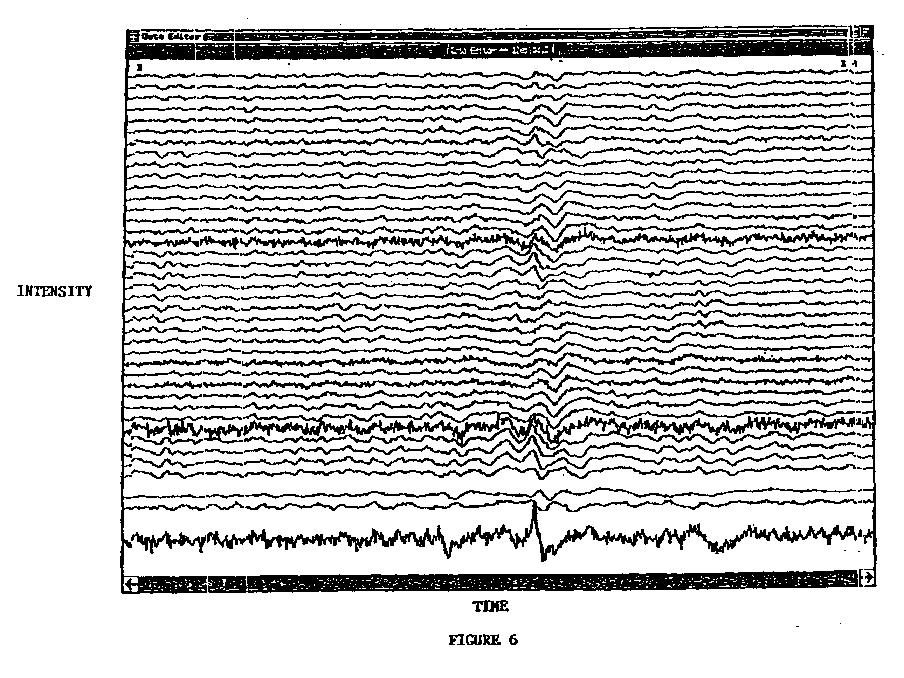

Figures 6-8 illustrate some of the results of this assessment. Figure 6 presents the signal magnitude as a function of time for the 37 channels of biomagnetic data (the top 37 lines), 2 channels of EEG data, and the spatially filtered analysis of the information (bottom line) for a specific selected location within the brain. In the central portion of the figure, the occurrence of an event can be discerned from some of the channels, but its magnitude and time cannot readily be determined. The spatially filtered result clearly shows the location in time of the event as a major peak. Spatial filtering thus is useful in ascertaining the fact of an event whose presence is difficult to determine otherwise in the measured data.

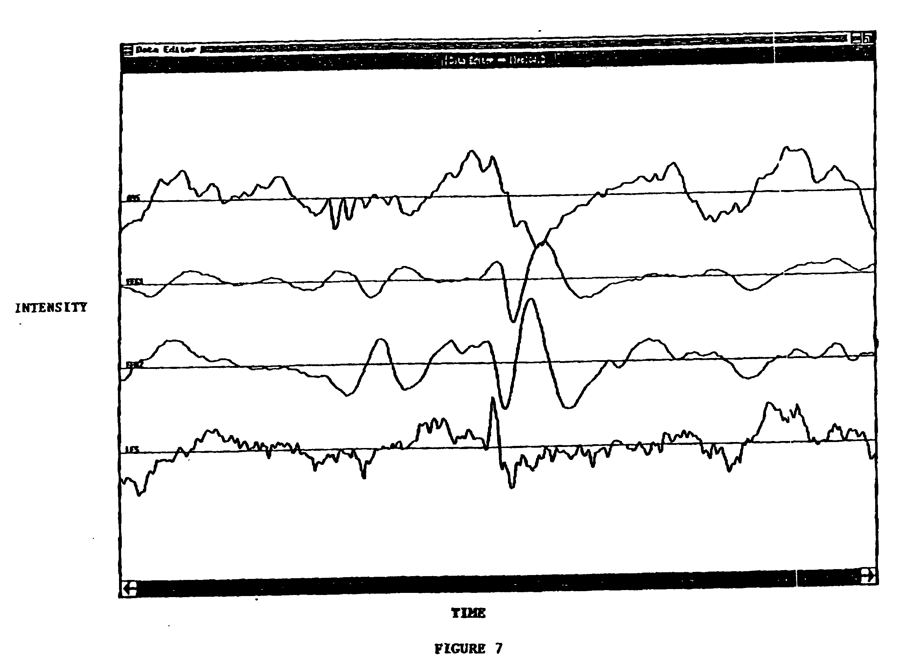

Figure 7 also illustrates signal magnitude as a function of time, in this case on expanded scales and presenting only the clearest of the biomagnetic signal channels, channel 35 (the top line), and the two EEG channels (middle two lines), together with the spatial filtering result from analysis of all of the data (bottom line). The fact of an event is visible in all of the curves, but its occurrence is most clearly defined in the spatial filtering calculation. The clearer definition of the peak of the event results in a different conclusion as to the time at which the event apparently occurs, than would be reached from the measured data. This result suggests that spatial filtering can be used to determine not only the location within the brain, but also the time of occurrence of the event, more precisely than previously possible. Since events at one location within the brain propagate to other locations or trigger other events, both the exact location and time of the event are of significance.

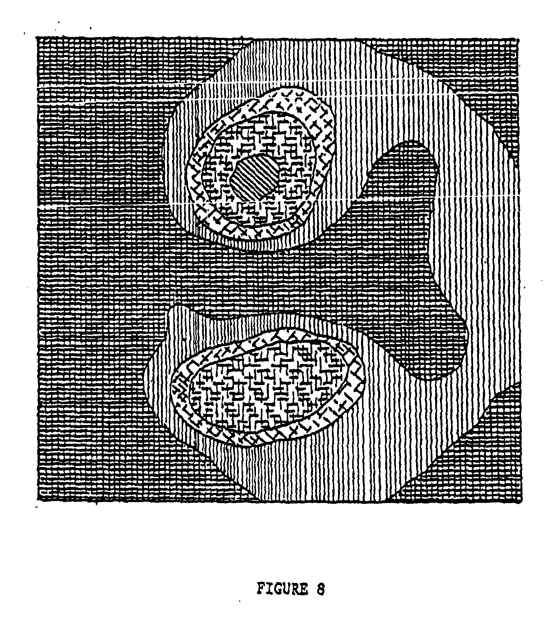

Another approach to analyzing data using spatial filtering is to prepare a spatial map of intensity. The mapping is accomplished by using a single set of measured data, and then performing repeated spatial filtering calculations to determine the signal arising at each location of a raster of points within the brain. The results of the analysis are most readily visualized as a false color or gray scale image of the event in space at a moment in time. To do this, the signal magnitudes are associated with color ranges or a gray scale, and presented on a monitor or plotter. Figure 8 is such a plot of data taken during an epileptic episode, presented in a five-color range. (Greater spatial resolution is attained with more colors.) The externally measured signals are seen to arise from two spatially distinct but apparently associated locations within the brain. This detailed structure of the event is not evident from visual inspection of the 37 channels of data, nor would it be determined from conventional dipole modeling of the event based upon the measured data. By making a series of plots such as Figure 8, at a series of times before, during, and after the episode, the spatial and temporal evolution of the event within the brain can be determined and presented as a "movie". These results are of interest to the researcher to determine the origins and participating regions of an event as it evolves through time, and also to the doctor seeking a method of treatment.

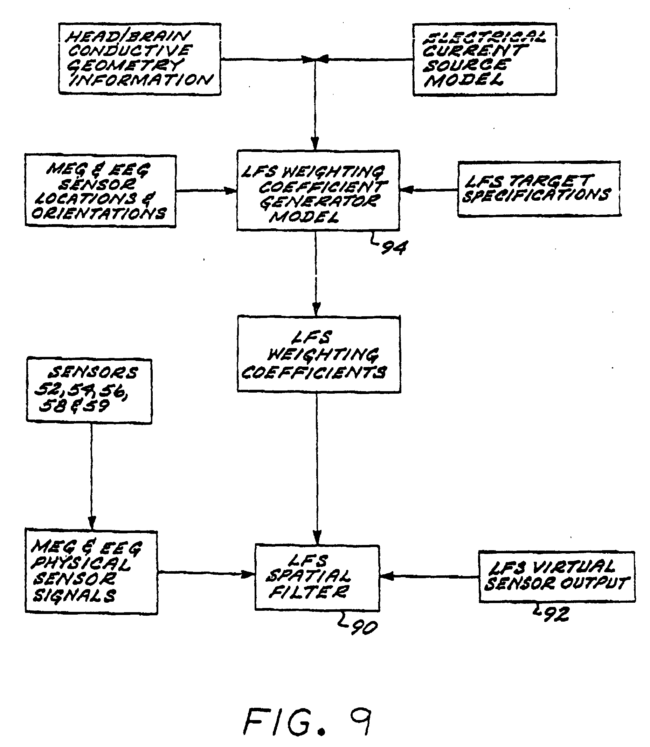

Another approach to the determination of weighting coefficients is illustrated in Figure 9. The physically measured sensor signals and the calculated weighting coefficients are supplied to the multiplying and adding functions, which are there termed a spatial filter 90. The present approach is a filter that selectively isolates and analyzes signals that are consistent with signals arising from particular volumes of the brain ("sources"). The result is the virtual sensor output 92, which can then be studied with respect to other virtual output signals, or with respect to physiological events or stimuli.

In this approach, the calculation of the weighting factors w is made using a mathematical model of the interaction of magnetic sources in the brain and external sensors. The results of these analyses are manifested in the form of the weighting coefficients that are selected.

The weighting coefficients are calculated from a mathematical model of the interaction between the magnetic activity and structure of the brain and external sensors, indicated at numeral 94. A model of the brain, with the attendant assumptions, is required in this approach. The physical laws governing the electrical potentials and magnetic fields due to current flow in a volume conductor are known, and can serve to compute potentials and fields using a modeling approach to the head. Considerations required for most realistic mathematical models of the brain include those shown as input to the model 94, including, but not limited to, the sensor locations, orientations, and lead fields, the geometry and response to magnetic fields of the brain and skull, the conducting properties of the brain and skull, the nature of the current sources in the brain, and the geometry and orientation of the target source.

The nature of the interaction of an external sensor with a source within the head requires knowledge or the source field and the sensitivity pattern of the sensor, but also requires an understanding of the media and the effect of the media upon the electromagnetic field. The human head, which is a shaped brain mass within a skull of complex shape, has been modeled in various ways. In one of the simplest, the effect of the skull is largely ignored. In another, the skull is considered, but idealized as a sphere. The present lead field synthesis approach can be utilized with any of such models, which provide the weighting factors to be used.

The preferred model at the present time is a finite mesh model, wherein the outer shape of the skull is measured and digitized. The inner surface of the skull is obtained from this digitization by subtracting the approximate thickness of the skull itself from the measured points. The inner surface provides the boundary for an assumed homogeneous brain contained within the skull. In this model, the current source is described as an elementary current dipole. This model provides useful results, and can be run on computers that are readily available as this patent application is written. More sophisticated finite element models are known and are generally more accurate, but are presently less preferred because of the excessively long computing time required.

It is to be understood that this embodiment of the lead field synthesis approach is not dependent upon the use of any particular model. It is expected that, as new models are developed and greater computational power becomes available, even more sophisticated models will be utilized. In those cases, the weighting coefficients may change from those of the finite mesh model, but are still used as the linear combination factors for combining the outputs of the sensors of an array.

Continuing with the development of the finite mesh model, the solution for the magnetic field due to a primary current source within a bounded homogeneous conductor is found in the publication of F. Grynszpan and D.B. Geselowitz, "Model Studies of the Magnetocardiogram", Biophysics Journal, vol. 13, pages 911-925(1979). Rephrasing their results for a current dipole source, the total magnetic field B(r), using the electrical potential at the surface of the object, $V(r_s)$ and the conductivity $\sigma$:

$$G_i = \vec{B}(\vec{r}) = \vec{B_o}(\vec{r}) - \sigma \frac{\mu_o}{4\pi} \int_S V(\vec{r_s}) n(\vec{r_s}) \times \frac{\vec{r} - \vec{r_s}}{|\vec{r} - \vec{r_s}|^3} dS$$

$\vec{B}_o$, the magnetic field at measuring point $\vec{r}$ produced by a current dipole $\vec{Q}$ at $\vec{r}_o$ within an infinite homogeneously conducting space, is given by

$$\vec{B_o}(\vec{r}) = \frac{\mu_o}{4\pi} \vec{Q} \times \frac{\vec{r} - \vec{r_s}}{|\vec{r} - \vec{r_s}|^3} dS$$

The potential on surface S is

$$V(\vec{r_s}) = 2 V_o(\vec{r_s}) + \frac{1}{2\pi} \int_S V(\vec{r_s}') d\Omega(\vec{r_s}')$$

where $d\Omega(\vec{r}_s')$ is the solid angle subtended at $\vec{r}_s$ by surface element $dS(\vec{r}_s)$, and $V_o$ is the potential within an infinite homogeneously conducting space due to a current dipole $\vec{Q}$:

$$V_o(\vec{r}) = \frac{1}{4\pi\sigma} \vec{Q} \cdot \frac{\vec{r} - \vec{r_o}}{|\vec{r} - \vec{r_o}|^3}$$

As shown by M.S. Hamalainen and J. Sarvas in their publication "Feasibility of the Homogeneous Head Model in the Interpretation of Neuromagnetic Fields", Phys. Med. Biol., vol. 32, pages 91-97 (1987), if the boundary is represented by a finite mean consisting of n triangular elements, the first-presented equation becomes:

$$\vec{B}(\vec{r}) = B_0(r) - \frac{\mu_0 \sigma}{4\pi} \sum_{i=1}^{n} V(\vec{c}_i) n(\vec{c}_i) \frac{\vec{r} - \vec{c}_i}{|\vec{r} - \vec{c}_i|^3} \Delta S_i$$

where $\vec{c}_i$ is the center point of a triangular element, n is the outward-pointing normal vector, and $\Delta S_i$ is the surface of the ith triangular surface element.

Similarly, the equation for $V(\vec{r}_s)$ may be expressed in discrete form by letting $w_{ij}$ be the solid angle subtended by the jth triangular element at center point $c_i$. The potential at each facet is

$$V(\vec{c}_i) \simeq 2V_0(\vec{c}_i) + \frac{1}{2\pi} \sum_{\substack{j=1 \\ j \neq i}}^{n} w_{ij} V(\vec{c}_j) \qquad i = 1,2\ldots n$$

This equation is solved after transformation of the surface element potentials with a set of linear equations

$$\alpha_i = \sum_{j=1}^{n} A_{ij} V_j \qquad i = 1,2,\ldots n$$

$$\alpha_i = 2V_0(\vec{c}_i), \qquad V_j = V(\vec{c}_j),$$

$$A_{ij} = \begin{cases} -\frac{1}{2\pi} w_{ij} & \text{if } i \neq j \\ 1 & \text{if } i = j \end{cases}$$

A unique solution is provided by replacing $A_{ij}$ with $A'_{ij} + 1/n$. The system of equations is solved by matrix inversion, and the surface potentials are substituted back into the discrete $B_r$ equation.

Different models may be developed to calculate the weighting coefficients for several reasons. First, different models may be applicable to different parts of the brain. Second, the models themselves may require refinement as to assumptions used and mathematical techniques used to solve them. As with many theories, as the model becomes more complex and realistic, its use may be limited by the mathematical techniques available for its solution.

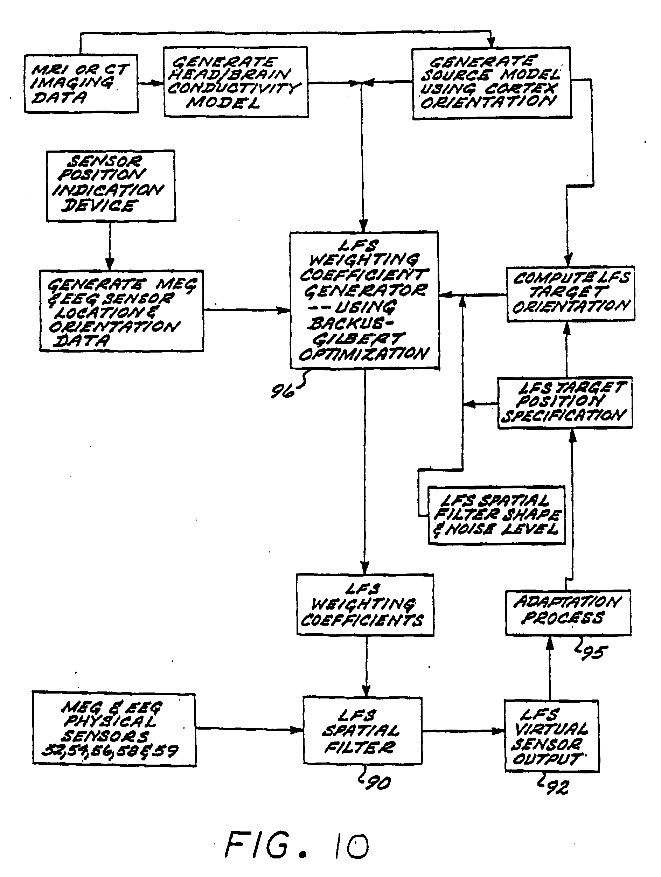

Some further development of the lead field synthesis methodology is presented in Figure 10. To provide positioning information in an automated manner, the position indication device of the '355 patent is used. Direct imaging data from other sources is used in the geometrical analysis of the head. Additionally, an adaptive analysis capability is provided, numeral 95. That is, the computer can alter the model used, the assumptions of the model, or the values used in the calculation, to determine whether these changes result in a more consistent result.

The augmented model further provides for a Backus-Gilbert optimization, numeral 96. A fundamental problem in ascertaining the signal associated with a particular location is that the number of potential signal and noise sources in the brain is far greater than the number of sensors that may be arrayed external to the brain to measure those signals. There is therefore always some uncertainty of the signal associated with a target location in light of brain and instrument noise.

The theory of Backus and Gilbert relates the selectivity of a solution to the noise experienced by the sensors. A review of this theory is found in the publication by R.L. Parker, "Understanding Inverse Theory", Ann. Rev. Earth Planet. Sci., vol. 5, pages 35-64 (1977). The methodology effectively rotates the spatial filter vector obtained from the forward solution described above, away from the interfering sources such that the projection of the noise sources onto the filter vector is minimized, while maintaining as large a projection for the target source as is possible.

Developing the Backus-Gilbert theory in a three-dimensional model, the optimum weight vector $\vec{W}$ is found by solving the matrix minimization

$$\min_{\vec{W}} \vec{W}^T A \vec{W}$$

subject to

$$\vec{W}^T B \vec{W} = 1$$

where

$$A_{ij} = \int_{\Lambda} G_i(\vec{r}_o) G_j(\vec{r}_o)(\vec{r}_o - \vec{r}_t)^2 d\Lambda$$

and

$$B_{ij} = \int_{\Lambda} G_i(\vec{r}_o) G_j(\vec{r}_o) d\Lambda$$

$r_o$ is any point within the head or body, and $r_t$ is the target location, the region of particular interest. Using a Lagrange multiplier and differentiating with respect to each of the variables leads to a simple matrix equation for the unknown vector of weights W. These weights are used as the multipliers, as for example the multipliers $w_{52}$, $w_{54}$, $w_{56}$, $w_{58}$, and $w_{59}$ of Figure 5.

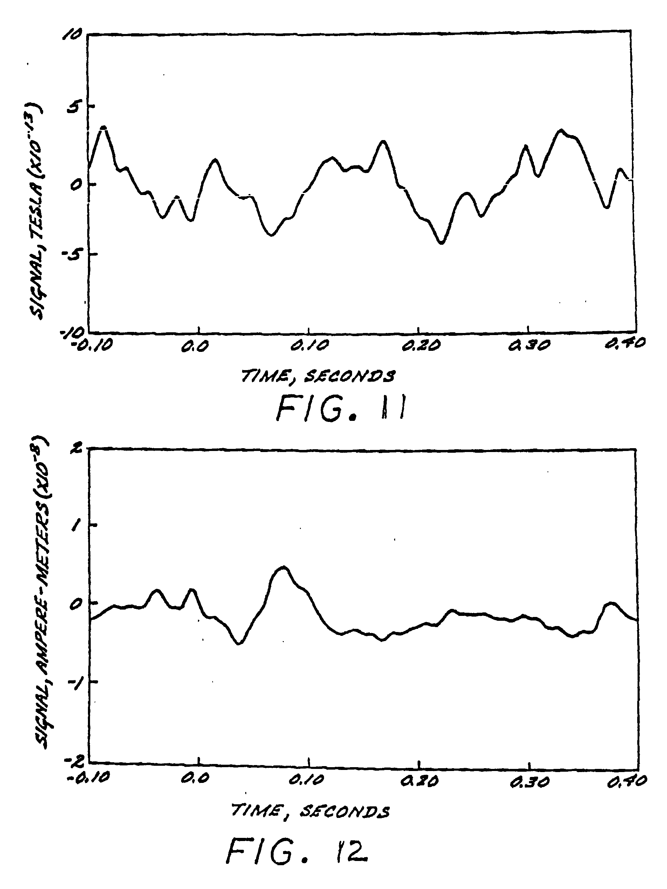

The approach described in the preferred embodiment was applied to the analysis of recordings of brain signals made with a 14-sensor biomagnetometer. An experiment was conducted in which a person's brain response to an external auditory stimulus was measured by the 14 magnetic sensors and recorded. Figure 11 is an exemplary output signal from one of the 14 physical sensors, which in this case was positioned over one of the extrema and therefore thought to be optimally positioned. (The other 13 physical sensors also produced output signals as a function of time which were recorded, but these signals are not reproduced here.) Using the brain coordinates determined for the response to an auditory stimulus, a spatial filter was computed using the approach of the preferred embodiment, and applied to the 14 output signals.

Figure 12 is the computed virtual signal, calculated as the weighted sum of the 14 individual sensor output signals. The response to the auditory stimulus, which was applied at 0.0 seconds, is not distinguishable from the brain noise in the physical sensor trace, Figure 11, nor was it distinguishable in any of the other physical sensor traces. However, the brain response can be dearly discerned in the virtual sensor trace, Figure 12. This result demonstrates that brain noise has been sufficiently suppressed by the present approach that source signals of interest can be discerned. With this capability to measure the response of the brain to the external stimulus, it is then possible to conduct experiments that determine the change in response of the brain to changes in the auditory signal, repetition of the auditory signal, or other factors under study.

The lead field synthesis or spatial filtering technique provides a valuable new approach for analyzing the magnetic signals (or electrical signals, with the appropriate sensors) generated within the human body, and particularly within the brain or the heart. The approach of the invention is used to analyze data collected from the sensors, to calculate the behavior of a source of interest while minimizing the effects of other sources within and also external to the brain. Although a particular embodiment of the invention has been described in detail for purposes of illustration, various modifications may be made without departing from the spirit and scope of the invention.

**Claims**

1. A process for sensing and evaluating signals emanating from within the body of a subject, comprising the steps of:

   providing an array of field sensors disposed external to the body of a subject at known locations;
   measuring a signal strength detected by each of said sensors;
   multiplying the signal strength measured by each of said sensors times a weighting coefficient for that sensor:
   **characterized in** the steps of selecting said weighting coefficient to focus the sensitivity of the array of field sensors on a selected location within the body of the subject, to determine a virtual sensor contribution for each of said sensors; and
   adding together the virtual sensor contribution of each of said sensors, to define a virtual sensor signal;
   wherein said weighting coefficient for each of said sensors is determined at least in part by the signal strengths detected by said array of sensors.

2. The process of claim 1, wherein the weighting coefficients $\vec{W}$ are calculated in matrix form as

$$\vec{W} = [C_{ij}]^{-1}[\vec{G_T}]/[\vec{G_T}] \cdot [C_{ij}]^{-1}[\vec{G_T}]$$

   where $C_{ij}$ is the covariance matrix of the measured signals of each of said sensors and $\bar{G}_T$ is the sensitivity matrix for each sensor, at a particular point.

3. The process according to one of the preceding claims, wherein each weighting coefficient is determined by the steps of characterizing the geometry of the portion of the body being measured, characterizing the nature of the magnetic fields emanating from possible sources in the body, characterizing the medium through which the magnetic fields propagate, formulating a mathematical model of the response of the array of electromagnetic sensors to a source within the body, and calculating the weighting coefficients from the model.

4. The process of claim 3, wherein the weighting coefficient for each sensor is selected using the mathematical model to optimize the contribution to the virtual signal of a target source, relative to signals not produced by the target source.

5. The process of claim 3 or 4, wherein the mathematical model is a finite mesh model.

6. The process according to one of the preceding claims, including the additional step, after the step of adding, of repeating the steps of multiplying and adding for a plurality of different points within the body of the subject.

7. The process of claim 6, including the additional step, after the step of repeating, of displaying the virtual sensor signals determined for the points in space on a spatial plot.

8. The process of claim 6 or 7, including the additional step, after the step of repeating, of repeating the steps of measuring, multiplying, and adding for a signal determined at a different time.

9. The process according to one of the preceding claims, wherein each of said sensors is a magnetic field sensor and/or an electric field sensor.

10. The process according to one of the preceding claims, wherein the sensors are placed proximately to the head of the subject and/or proximately to the chest of the subject.

11. The process according to one of the preceding claims, including, after the step of adding, the additional step of correlating the virtual sensor signal with a physiological activity.

**Patentansprüche**

1. Verfahren zum Aufnehmen und Bewerten von vom Inneren des Körpers eines Subjekts ausgesandten Signalen, mit folgenden Verfahrensschritten :

Bereitstellen einer Anordnung von Feldsensoren, welche außerhalb des Körpers eines Subjekts an bekannten Positionen vorgesehen sind;
Messen einer durch jeden der Sensoren detektierten Signalstärke;
Multiplizieren der von jedem Sensor gemessenen Signalstärke mit einem Gewichtungskoeffizienten für den betreffenden Sensor;

**gekennzeichnet durch** folgende Verfahrensschritte:

Auswählen des Gewichtungskoeffizienten, um die Empfindlichkeit der Anordnung von Feldsensoren auf eine ausgewählte Position innerhalb des Körpers des Subjekts zu fokusieren, um einen virtuellen Sensor-Beitrag für jeden der Sensoren zu bestimmen; und
Zusammenaddieren des virtuellen Sensor-Beitrags jedes der Sensoren, um ein virtuelles Sensorsignal zu definieren; wobei der Gewichtungskoeffizient für jeden der Sensoren wenigstens zum Teil anhand der Signalstärken, die von der Anordnung von Sensoren ermittelt wurden, bestimmt wird.

2. Verfahren nach Anspruch 1, **dadurch gekennzeichnet, daß** die Gewichtungskoeffizienten $\vec{W}$ in Matrixform berechnet werden als

$$\vec{W} \;=\; [C_{ij}]^{-1}[\vec{G_T}] \,/\, [\vec{G_T}] \star [C_{ij}]^{-1}[\vec{G_T}]$$

wobei $C_{ij}$ die Kovarianz-Matrix der gemessenen Signale jedes der Sensoren und $\vec{G_T}$ die Empfindlichkeits-Matrix -Matrix für jeden Sensor an einem bestimmten Punkt ist.

3. Verfahren nach einem der vorstehenden Ansprüche, **dadurch gekennzeichnet, daß** jeder Gewichtungskoeffizient bestimmt wird durch die Verfahrensschritte

Charakterisieren der Geometrie des gemessenen Körperbereichs,
Charakterisieren der Natur der von möglichen Quellen im Körper ausgesandten magnetischen Felder,
Charakterisieren des Mediums, welches die magnetischen Felder durchsetzen,
Formulieren eines mathematischen Modells der Antwort der Anordnung elektromagnetischer Sensoren auf eine Quelle innerhalb des Körpers, und
Berechnen der Gewichtungskoeffizienten anhand des Modells.

4. Verfahren nach Anspruch 3, **dadurch gekennzeichnet, daß** der Gewichtungskoeffizient für jeden Sensor unter Verwendung des mathematischen Modells ausgewählt wird, um den Beitrag zu dem virtuellen Signal einer Ziel-Quelle relativ zu Signalen, die nicht von der Ziel-Quelle stammen, zu optimieren.

5. Verfahren nach Anspruch 3 oder 4, **dadurch gekennzeichnet, daß** das mathematische Modell ein Finite-Maschen-Modell (finite mesh model) ist.

6. Verfahren nach einem der vorstehenden Ansprüche, welches nach dem Verfahrensschritt des Addierens den zusätzlichen Verfahrensschritt des Wiederholens der Schritte des Multiplizierens und Addierens für eine Mehrzahl verschiedener Punkte innerhalb des Körpers des Subjekts umfaßt.

7. Verfahren nach Anspruch 6, welches nach dem Verfahrensschritt des Wiederholens den zusätzlichen Verfahrensschritt des Anzeigens der für die Raumpunkte ermittelten virtuellen Sensorsignale in Form eines räumlichen Plots umfaßt.

8. Verfahren nach Anspruch 6 oder 7, welches nach dem Verfahrensschritt des Wiederholens den zusätzlichen Verfahrensschritt des Wiederholens der Schritte des Messens, Multiplizierens und Addierens für ein zu einer anderen Zeit bestimmtes Signal umfaßt.

9. Verfahren nach einem der vorstehenden Ansprüche, **dadurch gekennzeichnet, daß** jeder der Sensoren ein magnetischer Feldsensor und/oder ein elektrischer Feldsensor ist.

**10.** Verfahren nach einem der vorstehenden Ansprüche, **dadurch gekennzeichnet, daß** die Sensoren nahe dem Kopf des Subjekts und/oder nahe dem Brustbereich des Subjekts angeordnet werden.

**11.** Verfahren nach einem der vorstehenden Ansprüche, welches nach dem Verfahrensschritt des Addierens den zusätzlichen Verfahrensschritt des Korrelierens des virtuellen Sensorssignals mit einer physiologischen Aktivität umfaßt.


**Revendications**

**1.** Procédé de détection et d'évaluation de signaux provenant de l'intérieur du corps d'un sujet, comprenant les étapes suivantes:

mise en place d'un réseau de capteurs de champ disposés à l'extérieur du corps d'un sujet en des emplacements connus;
mesure de l'intensité d'un signal détecté par chacun desdits capteurs;
multiplication de l'intensité du signal mesuré par chacun desdits capteurs par un coefficient de pondération pour ce capteur;

**caractérisé par** les étapes de

sélection dudit coefficient de pondération pour concentrer la sensibilité du réseau de capteurs de champ sur un emplacement sélectionné à l'intérieur du corps du sujet, pour déterminer la contribution d'un capteur virtuel pour chacun desdits capteurs ; et
d'addition globale de la contribution du capteur virtuel de chacun desdits capteurs, pour définir un signal de capteur virtuel ;
dans lequel ledit coefficient de pondération pour chacun desdits capteurs est déterminé au moins en partie par les intensités des signaux détectés par ledit réseau de capteurs.

**2.** Procédé selon la revendication 1, dans lequel les coefficients de pondération W sont calculés sous forme matricielle, de la manière suivante:

$$\vec{W} = [C_{ij}]^{-1} \, [\vec{G_T}] \, / \, [\, \vec{G_T}\,] \cdot [C_{ij}]^{-1} \, [\vec{G_T}]$$

où $C_{ij}$ est la matrice de covariance des signaux mesurés de chacun desdits capteurs, et $\vec{G_T}$, la matrice de sensibilité pour chaque capteur, en un point particulier.

**3.** Procédé selon l'une quelconque des revendications précédentes, dans lequel chaque coefficient de pondération est déterminé par les étapes de caractérisation de la géométrie de la partie du corps concernée par la mesure, de caractérisation de la nature des champs magnétiques provenant de sources possibles dans le corps, de caractérisation du milieu dans lequel les champs magnétiques se propagent, de formulation d'un modèle mathématique de la réponse du réseau de capteurs électromagnétiques à une source dans le corps, et de calcul des coefficients de pondération à partir du modèle.

**4.** Procédé selon la revendication 3, dans lequel le coefficient de pondération pour chaque capteur est sélectionné en utilisant le modèle mathématique pour optimiser la contribution au signal virtuel d'une source cible, par rapport aux signaux non produits par la source cible.

**5.** Procédé selon la revendication 3 ou la revendication 4, dans lequel le modèle mathématique est un modèle maillé fini.

**6.** Procédé selon l'une quelconque des revendications précédentes, comprenant l'étape supplémentaire, après l'étape d'addition, de répétition des étapes de multiplication et d'addition pour une pluralité de différents points à l'intérieur du corps du sujet.

**7.** Procédé selon la revendication 6, comprenant l'étape supplémentaire, après l'étape de répétition, d'affichage des signaux du capteur virtuel déterminés pour les points dans l'espace sur un tracé spatial.

8. Procédé selon la revendication 6 ou la revendication 7, comprenant l'étape supplémentaire, après l'étape de répétition, de répétition des étapes de mesure, de multiplication, et d'addition pour un signal déterminé à un moment différent.

9. Procédé selon l'une quelconque des revendications précédentes, dans lequel chacun desdits capteurs est un capteur de champ magnétique et/ou un capteur de champ électrique.

10. Procédé selon l'une quelconque des revendications précédentes, dans lequel les capteurs sont placés à proximité de la tête du sujet et/ou à proximité de la poitrine du sujet.

11. Procédé selon l'une quelconque des revendications précédentes, comprenant, après l'étape d'addition, l'étape supplémentaire de corrélation du signal du capteur virtuel avec une activité physiologique.

FIG.1

FIG.2

FIG.3

FIG.4

FIG. 5

INTENSITY

TIME

FIGURE 6

EP 0 477 434 B2

INTENSITY

TIME

FIGURE 7

FIGURE 8

FIG. 9

FIG. 10

FIG. 11

FIG. 12